# EUROPEAN PATENT SPECIFICATION

(11) **EP 0 598 116 B1**
(45) Date of publication and mention of the grant of the patent: **24.07.1996**
(21) Application number: 90917270.2
(22) Date of filing: 26.11.1990
(51) Int. Cl.: A61K 9/107, A61K 31/71

(54) **FAT EMULSION**
FETTEMULSION
EMULSION GRASSE

(30) Priority: 27.11.1989 JP 308622/89
(43) Date of publication of application: 25.05.1994
(73) Proprietor: NIPPON SHINYAKU COMPANY, LIMITED, Minami-ku Kyoto-shi Kyoto 601 (JP)
(72) Inventor: SEKI, Junzo, Osaka 567 (JP); USHIMARU, Kouichi, Kyoto-shi Kyoto 602 (JP); SUGIYAMA, Makoto, Kyoto-shi Kyoto 607 (JP)
(74) Representative: Kügele, Bernhard
(86) International application number: JP9001531
(87) International publication number: WO9107973

(56) References cited:
- EP-A- 0 202 837
- EP-A- 0 211 258
- EP-A- 0 296 845
- WO-A-88/07365
- JP-A- 1 160 915
- JP-A-58 222 014
- JP-A-59 141 518
- JP-A-59 216 820
- JP-A-61 291 512
- JP-A-63 066 213
- JP-A-63 208 515
- CHEMICAL ABSTRACTS Abstract No. 113 (16): 1384336b, Chemotherapy (Tokyo), 38 (6), 548-51 (1990) MIYAZAKI TAKASHIGE, KOHNO SHIGERU, YASUOKA AKIRA, MAESAKI SHIGEFUMI, YAMADA HIROSHI, SASAYAMA KAZUO, DOHTSU YASUMASA, YAMAGUCHI KEIZO HIROTA MASAKI et al.: "A lipid emulsion formulation of amphotericin B for the treatment of murine candidiasis and cryptococcosis".
- CHEMICAL ABSTRACTS Abstract No. 111 (18): 160102y, Int. J. Pharm., 54 (1), 41-9 (1989), ILLUM L., WEST P., NOTTINGHAM C., DAVID S.S.: "The effect of stabizing agents on the organ distribution of lipid emulsions"
- CHEMICAL ABSTRACTS Abstract No. 109 (24): 215813x, Ann. N. Y. Acad. Sci., 507 (Biol, Approaches Controlled Delivery Drugs), 75-88 (1987), DAVIS STANLEY S., WASHINGTON CLIVE, WEST PHILIP, ILLUM LISBETH, LIVERSIDGE GARRY, STERNSON LARRY, KIRSH RICHARD: "Lipid emulsions as drug delivery systems".
- Chemical Abstracts Abstract No. 109 (20): 176197c, Int. J. Pharm., 46 (1-2), 25-30 (1988), WASHINGTON C., TYLOR S.J., CAVIS S.S.: "The structure of colloidal furmulations of amphotericin B".
- CHEMICAL ABSTRACTS Abstract No. 109 (4): 27563s, J. Pharm. Pharmacol., 40 (5), 325-8 (1988), FORSTER D., WASHINGTON C., DAVIS S.S.: "Toxicity of solubilized and colloidal amphotericin B formulations to human erythrocytes".

## Description

### FIELD OF THE INVENTION

The present invention relates to a novel fat emulsion comprising as a main ingredient a polyene antifungal antibiotic.

### BACKGROUND OF THE INVENTION

About 30 years have passed since polyene antifungal antibiotics represented by Amphotericin B were developed; even now, polyene antifungal antibiotics are useful as important antifungal agents which can be generally administered and by which reliable effects can expect.

However, their clinical use is extremely limited by serious side effects such as hemolytic toxicity and nephrotoxicity and hence, these antifungal agents encounter a problem that chemotherapy does not work satisfactorily.

In addition, when polyene antifungal antibiotics are applied in the form of injection, a surface active agent called sodium deoxycholate which is irritative and hemolytic must be used and an improvement in medical preparation has been desired.

In recent years, in order to alleviate these side effects, polyene antifungal antibiotics have been administered either as liposome preparations comprising phospholipids or as fat emulsion preparations obtained by emulsifying soybean oil with a small quantity of phospholipid (Szoka, F.C. Jr., et al., Antimicrobial Agents and Chemotherapy, 31, 421-429, 1987 [hereafter referred to as "Publication No. 1"], Kirsh, R. et al., Journal of Infectious Diseases, 158, 1065-1070, 1988 [hereafter referred to as
"Publication No. 2"], Japanese Patent Application Laid-Open No. 66123/89 [hereafter referred to as "Publication No. 3"].

However, these various liposome preparations and fat emulsion preparations involve a serious defect that the preparations are hardly effective for alleviating nephrotoxicity which is the most serious problem in clinical use, although these preparations successfully reduce hemolytic toxicity possessed by the polyene antifungal antibiotics and alleviate acute toxicity.

In addition, these various liposome preparations and fat emulsion preparations are characterized by undergoing phagocytosis with macrophage, etc. gathered at the infected site. However, in evaluation on a general level, a defect encounters that most of the drug administered undergo phagocytosis by reticuloendothelial cells represented by liver and spleen to migrate and true transfer of the drug into the infected site is not necessarily effective.

On the other hand, in view of manufacturing medical preparations and safety of the preparations, liposome preparations are questionable in production in an industrial scale. The liposome preparations also involve a serious problem in stability during storage because of increase in particle size due to aggregation.

Fat emulsions which have been hitherto used clinically as nutrient supplementation fluid have been used as injection preparations of various drugs, and utility of these preparations is known. However, it was difficult to apply fat emulsion preparations to polyene antifungal antibiotics because such preparations encounter serious problems in preparing emulsions and in stability of the preparations due to amphiphatic property of the drugs and poor solubility in soybean oil, etc. It was thus difficult to overcome the problem.

In general, an administered drug migrates and is distributed in the body depending on the property inherent possessed by the drug molecule. When a part of the drug reaches the site to be acted, its pharmaceutical effect is exhibited. In this case, it is preferred that the drug be concentrated only at the site necessary for exhibiting the pharmaceutical effect. In general, however, a drug is widely distributed over the entire body and migrates also to the sites which do not require the drug; this sometimes causes side effects. Therefore, it is important and necessary to improve a disposition of a drug in the body.

In view of the situations described above, the present inventors have made extensive investigations on preparation forms for administration which can reduce the hemolytic toxicity and nephrotoxicity and facilitate excellent migration of a drug toward infected sites without adversely affecting the mechanism itself of pharmacological action (antifungal action) of polyene antifungal antibiotic on a molecular level. As a result, the present inventors have finally reached the present invention.

An object of the present invention lies in reducing nephrotoxicity of polyene antifungal antibiotics which is definitely the most serious problem in clinical field and providing medical preparations which enable to administering an effective dose of a drug safely without any concern of inducing a serious nephrotic disturbance.

### DISCLOSURE OF INVENTION

A characteristic feature of the present invention resides in limiting a relative proportion of each ingredient of a polyene antifungal antibiotic, a simple lipid, phospholipid and water in the composition, upon preparing a fat emulsion comprising the polyene antifungal antibiotic as a main ingredient.

In the present invention, the polyene antifungal antibiotic is incorporated in an amount of 0.005 to 5% (w/v) based on the total weight of the fat emulsion.

In the present invention, the simple lipid is incorporated in an amount of 0.5 to 30% (w/v) based on the total weight of the fat emulsion.

In the present invention, the phospholipid is incorporated in an amount of 0.15 to 2 times in a weight ratio based on the simple lipid described above.

Water which is the ingredient of the present invention is incorporated in a suitable amount.

The fat emulsion of the present invention is characterized in its ingredients and proportion of the ingredient, as compared to conventionally known liposome preparations or fat emulsions. That is, the fat emulsion of the present invention is greatly different from liposome in the construction and structure in that the emulsion is obtained by emulsifying a simple lipid such as soybean oil, etc. with phospholipid. Furthermore, its phospholipid content is greatly different from conventional fat emulsions used as preparation forms of various drugs for intravenous administration.

By the above construction, the effects that could not be obtained by conventional liposome preparations and fat emulsions can be obtained. Hereafter the fat emulsion is described in detail.

The fat emulsion of the present invention does not contain emulsion particles of 1 µm or greater.

A mean particle diameter of the fat emulsion of the present invention is in the range of from 10 nm, inclusive, to less than 200 nm. This is because the emulsion particles readily migrate from blood vessel into the focus tissue at the site where vascular permeability is accentuated due to inflammatory reaction caused by infection with fungi, etc.

At such infected sites, many emulsion particles of the present invention selectively migrate from blood vessel into the focus tissue.
At the same time, the drug included in the emulsion particle also migrates into the focus. By such migration, the drug migrates selectively into the focus easily so that the drug concentration at the focus site increases, whereby the effect of the drug can be enhanced.

A mean particle diameter of the fat emulsion in accordance with the present invention is preferably 100 nm or less. The fat emulsion having such a diameter range is excellent in avoiding intake by the reticuloendothelial system.

In addition, where the fat emulsion of the present invention is administered, the disturbance noted with the polyene antifungal antibiotic against the kidney function is not recognized at all. When the fat emulsion of the present invention is applied, an amount of the polyene antifungal antibiotic migrated into kidney can be extremely minimized and as the result, it is considered that alleviation of kidney impairment can be achieved.

Another characteristic feature of the present invention resides in using finely divided, stable emulsion particles as a preparation form of the polyene antifungal antibiotic.

By dividing the antibiotic into fine particles, not only the effect described above but also the effect of maintaining blood concentration of the drug can be obtained by inhibiting non-specific uptake of the drug by the reticuloendothelial tissue or the like.

The polyene antifungal antibiotic is a relatively unstable drug and it is known that the polyene antifungal antibiotic is gradually decomposed in an aqueous solution. In the present invention, however, the polyene antifungal antibiotic is present in oil droplets of the lipid and hence, are present in such a state that the antibiotic is shielded from the surrounding environment. Therefore, enzymatic or non-enzymatic decomposition can be prevented so that stability of the drug is also improved.

As described above, the fat emulsion particles in accordance with the present invention are characterized by using the surface layer (for example, refined egg yolk lecithin) in large quantities in its proportion to the nucleus of the emulsion particles (for example, soybean oil), as compared to conventional fat emulsions using high calorie supplementation fluid comprising soybean oil and egg yolk lecithin which belongs to the prior art. By such a formulation, a stable fat emulsion of fine particles containing the polyene antifungal antibiotic can be obtained for the first time.

In the fat emulsion of the present invention, it is necessary to use phospholipid in an amount of 0.15 to 2 times that of the simple lipid.

By using the phospholipid in such an amount, the surface area at the part which becomes the core of the emulsion particle by finely dividing the emulsion increases to cover the core as the surface layer of the emulsion particles, whereby the amount of phospholipid required to stabilize the emulsion can be sufficiently supplied.

Furthermore, this means that the amount of phospholipid required for stably retaining the polyene antifungal antibiotic in the emulsion particles can be sufficiently supplied.

With the amount less than the lower limit described above, it is unavoidable that coarse particles are intermingled so that any stable emulsion containing the drug cannot be obtained. Where the phospholipid is used in an amount exceeding the upper limit, it is unavoidable that liposome particles are intermingled so that any uniform fat emulsion cannot be obtained.

It is required that the content of the polyene antifungal antibiotic in the present invention be 5% (w/v) or less.

Examples of the simple lipid used in the present invention include neutral lipids such as refined soybean oil, cotton seed oil, rape seed oil, sesame oil, corn oil, peanut oil, safflower oil, triolein, trilinolein, tripalmitin, tristearin, trimyristin, triarachidonin, etc. Additional examples also include sterol derivatives such as cholesteryl oleate, cholesteryl linolate, cholesteryl myristate, cholesteryl palmitate, cholesteryl arachidate, etc.

Neutral lipids are relatively easily decomposed by a variety of lipase present in vascular endothelium, etc., whereas cholesterol derivatives undergo decomposition by these enzymes only with difficulty so that stability further increases in vivo. Therefore, the neutral lipids are preferable as the ingredients of the present invention.

Examples of the phospholipid which can be ued in the present invention include phospholipids derived from egg yolk, soybean, bovine, swine, etc. and phospholipids obtained purely synthetically or semi-synthetically. That is, examples include phosphatidylcholine, phosphatidylethanolamine, phosphatidylserine, phosphatidylinositol, phosphatidylglycerol, etc.

Additional examples include egg yolk phosphatidylcholine, soybean phosphatidylcholine dipalmitoyl phosphatidylcholine, dimyristoyl phosphatidylcholine, distearoyl phosphatidylcholine, dioleoyl phosphatidylcholine, dipalmitoyl phosphatidylglycerol, etc.
Hydrogenated products thereof may also be used. Among them, refined egg yolk lecithin is a preferred representative.

In order to impart surface charge to the emulsion particles, lipids having a charge such as stearylamine, dicetyl phosphate, phosphatidic acid, phosphatidylglycerol, etc. may also be used.

In producing the fat emulsion of the present invention, various methods for producing emulsions conventionally used heretofore may also apply as they stand. It is conventional that for example, all of the ingredients including the drug are sufficiently finely ground by using a pressure jet type homogenizer such as Manton-Gaulin type, etc., a microfluidizer, a ultrasonic homogenizer, etc. to form the fat emulsion of the present invention.

In the production, sterols, fatty acids or derivatives thereof, which are generally known as emulsifying aids or stabilizers and physiologically acceptable, may also be incorporated. Representative examples of these substances include cholesterol, oleic acid, etc.

The shape and particle diameter of the fat emulsion in accordance with the present invention can be readily confirmed by an electron microscope, a particle diameter analysis device of light scattering type, filtration through a membrane filter, etc.

In order to achieve a higher utilization value, other ingredients may also be added to the fat emulsion of the present invention. As such ingredients, additives, auxiliary substances and the like which are generally used for injection may be illustratively shown.
For example, there are an antioxidant, a preservative, a stabilizer, an isotonic agent, a buffering agent, and the like. The amounts required and the optimum amounts of these additives, auxiliary substances, etc. may be varied depending upon purpose.

The thus obtained fat emulsion of the present invention is sterilized (for example, sterilization by filtration, sterilization by high pressure steam, etc.), if necessary, and sealed in an ampoule together with nitrogen gas. In addition, the fat emulsion can be freeze-dried, if necessary. The freeze-dried fat emulsion of the present invention can be thawed by adding a suitable solution thereto.

The fat emulsion of the present invention is generally administered intravenously to human or various animals, for the purposes of treating or preventing antifungal infections or viral infections. In this case, it is necessary to sufficiently control the particle diameter, etc. of the emulsion particles.

In injection for intravenous application, it is known that when particles of 1 µm or more are intermingled, various toxic conditions generally appear. The fat emulsion of the present invention can be administered as injection intraarterially, intramuscularly, intramedullarly and subcutaneously, as in conventional fat emulsions. In addition, the fat emulsion of the present invention can be prepared into and used as eye drops, nose drops, oral agents, inhalations, urinary-bladder injections, suppositories, ointments, etc. Also in these cases, additives which are pharmaceutically acceptable bases, excipients, etc. may also be added to the fat emulsion of the present invention.

A dose of the fat emulsion in accordance with the present invention which is administered may be varied depending upon route for administration, preparation form, condition and purpose but the dose of 1 to 1000 ml/time is generally sufficient as the emulsion. A dose of the polyene antifungal antibiotic administered is in the range of 1 to 200 mg/time for adult.

As the polyene antifungal antibiotic which can be applied to the present invention, there are Amphotericin B methyl ester, Nystatin, Trichomycin, Pimaricin, etc., in addition to Amphotericin B.

According to the present invention, clinical utilization value of the polyene antifungal antibiotic can be markedly enhanced. As the effects of the present invention, the problems in the prior art are overcome and following effects are exhibited: (1) not only hemolytic toxicity possessed by polyene antifungal antibiotics but also nephrotoxicity, which is the serious problem to be solved, are markedly reduced; (2) migration of drugs into the focus is improved; (3) uptake by the reticuloendothelial system is prevented; (4) duration of blood concentration of drugs contained is realized; (5) stability during storage is ensured; (6) production costs are reduced, etc. These effects are obtained by the present invention for the first time.

The ingredients of the fat emulsion in accordance with the present invention are mainly medically acceptable lipids which have been hitherto used practically as medical drugs in clinical fields. Therefore, the fat emulsion of the present invention can be used extremely safely.

### BEST MODE FOR PRACTICING THE INVENTION

Hereinafter the present invention is described in more detail, with reference to examples relating to the production of the fat emulsion in accordance with the present invention. However, it is apparent that the present invention is not limited only to these examples.

### Preparation Example 1

After 3 mg of Amphotericin B, 0.5 g of refined soybean oil and 0.5 g of refined egg yolk lecithin are mixed with each other and dissolved in 100 ml of chloroform/methanol (l/l, v/v) mixture, the solvent is completely removed under reduced pressure using a rotary evaporator. Thereto is added 8 ml of isotonic phosphate buffer solution. The mixture is stirred with a homogenizer to form a coarse emulsion. Isotonic phosphate buffer solution is added to the emulsion to make the volume 10 ml. Thereafter the mixture is emulsified for 60 minutes under ice cooling, using a ultrasonic homogenizer (Branson Model 185) to obtain a fine fat microemulsion containing Amphotericin B having a mean particle diameter of 45 nm.

### Preparation Example 2

After 3 g of Amphotericin B, 50 g of refined soybean oil and 15 g of refined egg yolk lecithin are heated to about 60°C and mixed with each other, 500 ml of isotonic phosphate buffer solution is added to the mixture to form a coarse emulsion. The coarse emulsion is emulsified under high pressure using a Manton-Gaulin type homogenizer to obtain a fine fat microemulsion containing Amphotericin B having a mean particle diameter of 70 nm.

### Preparation Example 3

After 30 mg of Amphotericin B, 0.6 g of refined soybean oil and 0.5 g of refined egg yolk lecithin are mixed with each other and dissolved in 100 ml of chloroform/methanol (l/l, v/v) mixture, the solvent is completely removed under reduced pressure using a rotary evaporator. Thereto is added 8 ml of 0.24 M glycerine aqueous solution. The mixture is stirred with a homogenizer to form a coarse emulsion. After 0.24 M glycerine aqueous solution is added to the emulsion to make the volume 10 ml, the mixture is emulsified for 60 minutes under ice cooling, using a ultrasonic homogenizer (Branson Model 185) to obtain a fine fat microemulsion containing Amphotericin B having a mean particle diameter of 93 nm.

### Preparation Example 4

After 2 g of Amphotericin B, 20 g of refined soybean oil and 30 g of refined egg yolk lecithin are heated to about 60°C and mixed with each other, 100 ml of 0.24 M glycerine aqueous solution is added to the mixture. The mixture is stirred with a homomixer to obtain a coarse emulsion. The coarse emulsion is emulsified under high pressure using a microfluidizer to obtain a fine fat microemulsion containing Amphotericin B having a mean particle diameter of 20 nm.

### Preparation Example 5

After 1 mg of Amphotericin B, 0.5 g of cholesteryl oleate and 0.5 g of refined egg yolk lecithin are mixed with each other and dissolved in 100 ml of chloroform/methanol (l/l, v/v) mixture, the solvent is completely removed under reduced pressure using a rotary evaporator. Thereto is added 8 ml of 0.24 M glycerine aqueous solution. The mixture is stirred with a homogenizer to form a coarse emulsion. After 0.24 M glycerine aqueous solution is added to the emulsion to make the volume 10 ml, the mixture is emulsified for 60 minutes under ice cooling, using a ultrasonic homogenizer (Branson Model 185) to obtain a fine fat microemulsion containing Amphotericin B having a mean particle diameter of 55 nm.

### Preparation Example 6

After 3 mg of Amphotericin B, 0.5 g of refined soybean oil and 0.1 g of dimyristoyl phosphatidylglycerol are mixed with each other and dissolved in 100 ml of chloroform/methanol (l/l, v/v) mixture, the solvent is completely removed under reduced pressure using a rotary evaporator. Thereto is added 8 ml of 9% lactose aqueous solution. The mixture is stirred with a homogenizer to form a coarse emulsion. After 9% lactose aqueous solution is added to the emulsion to make the volume 10 ml, the mixture is emulsified for 60 minutes under ice cooling, using a ultrasonic homogenizer (Branson Model 185) to obtain a fine fat microemulsion containing Amphotericin B having a mean particle diameter of 48 nm.

### Preparation Example 7

After 3 mg of Amphotericin B, 0.5 g of refined soybean oil, 0.4 g of hydrogenated egg yolk lecithin and 0.1 g of cholesterol are mixed with each other and dissolved in 100 ml of chloroform/methanol (l/l, v/v) mixture, the solvent is completely removed under reduced pressure using a rotary evaporator. Thereto is added 8 ml of 9% lactose aqueous solution. The mixture is stirred with a homogenizer to form a coarse emulsion. After 9% lactose aqueous solution is added to the emulsion to make the volume 10 ml, the mixture is emulsified for 60 minutes under ice cooling, using a ultrasonic homogenizer (Branson Model 185) to obtain a fine fat microemulsion containing Amphotericin B having a mean particle diameter of 31 nm.

### Preparation Example 8

After 0.5 g of albumin is added to the drug compositions containing Amphotericin B obtained in Preparation Examples 1, 5 and 6, the composition is freeze-dried to give the freeze-dried preparation.

The results obtained by evaluating the properties of the drug compositions containing Amphotericin B in accordance with the present invention are shown below. In each test, commercially available Amphotericin B preparation, various liposome preparations containing Amphotericin B which belong to the prior art, and a conventional fat emulsion are used for the purpose of comparison. Details of each sample are described below.
Sample 1: Drug composition containing Amphotericin B in accordance with the present invention which is obtained in Preparation Example 1
Sample 2: Drug composition containing Amphotericin B in accordance with the present invention which is obtained in Preparation Example 3
Comparative Sample 1: Commercially available Amphotericin B preparation for injection (trademark: Fungizone (registered trademark), Squibb Japan)
Comparative Sample 2: Liposome preparation containing Amphotericin B which is prepared according to Publication No. 1, composed of dimyristoyl phosphatidylcholine : dimyristoyl phosphatidylglycerol = 7 : 3 in molar ratio and classified into multilamellar liposome
Comparative Sample 3: Liposome preparation containing Amphotericin B which is prepared according to Publication No. 1, composed of dimyristoyl phosphatidylcholine : dimyristoyl phosphatidylglycerol = 7 : 3 in molar ratio, obtained after ultrasonic treatment and classified into small unilamellar liposome
Comparative Sample 4: Liposome preparation containing Amphotericin B which is prepared according to Publication No. 1, composed of refined egg yolk lecithin, obtained after ultrasonic treatment and classified into small unilamellar liposome
Comparative Sample 5: Liposome preparation containing Amphotericin B which is prepared according to Publication No. 2, composed of refined soybean oil and refined egg yolk lecithin

### Test Example 1: Test on Hemolysis

Hemolytic action on purified rat erythrocyte was examined in vitro with respect to Sample 1 and Comparative Sample 1. Comparative Sample 1 showed marked hemolysis in an extremely low concentration (0.1 µg/ml or more) of Amphotericin B but Sample 1 hardly showed even in the concentration of 200 times or more. It is thus clear that the fat emulsions of the present invention greatly reduce hemolytic toxicity possessed by Amphotericin B itself, as in conventionally known liposome preparations and fat emulsion preparation.

### Test Example 2: Acute Toxicity (in vivo)

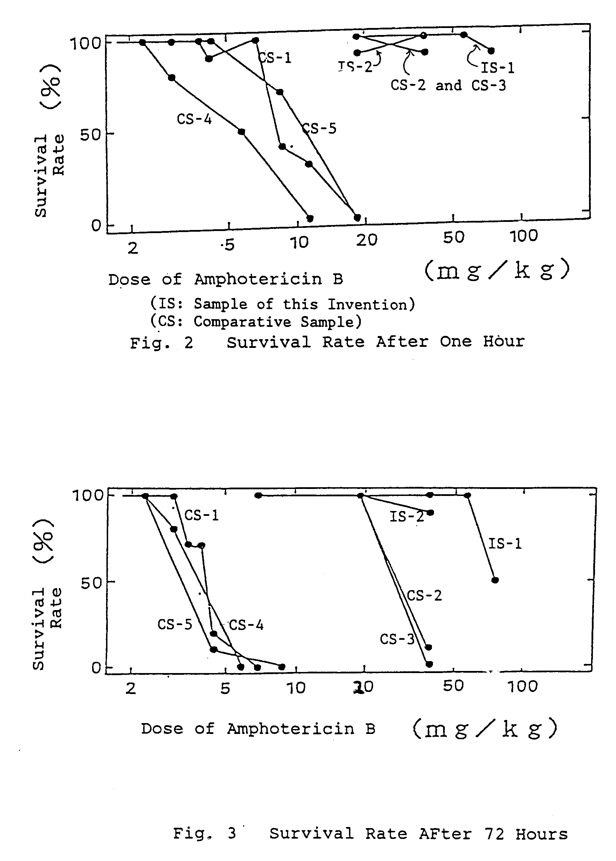

Using ddY strain male mice (weighing about 20 g) as experimental animals, each of Samples and Comparative Samples was intravenously administered from the tail vein to evaluate their acute toxicity. Figs. 2 and 3 show survival rates of the mice 1 and 72 hours after single administration.

The survival rate evaluated 1 hour after the administration which is shown in Fig. 2 demonstrates toxic evaluation mainly due to hemolysis possessed by Amphotericin B. Samples of the present invention tested all showed low toxicity. Among Comparative Samples, reduction in toxicity due to hemolysis was noted in Comparative Samples 2 and 3. However, alleviation of acute toxicity was not noted in Comparative Samples 1, 4 and 5.

The survival rate evaluated 72 hours after the administration which is shown in Fig. 3 demonstrates toxic evaluation mainly due to nephrotic toxicity possessed by Amphotericin B.
Samples of the present invention tested all showed extremely low toxicity. However, in all of Comparative Samples, toxicity appeared, indicating that nephrotoxicity is remarkable as compared to Samples of the present invention.

It is evident that in the fat emulsions of the present invention, the effects of reducing toxicity not only in the hemolytic toxicity observed immediately after administration but also especially in the toxicity evaluated 72 hours after administration which is considered to be due to nephrotoxicity are remarkable.

### Test Example 3: Amount of Drug in Kidney (distribution into kidney)

Using SD strain male rats (weighing about 250 g) as experimental animals, each of Samples of the present invention and Comparative Samples was intravenously administered through the tail vein. The dose was 1 mg/kg as Amphotericin B. 18 hours after administration, the kidney was removed and homogenized. Then the concentration of Amphotericin B in kidney was determined by high performance liquid chromatography. The results are shown in Table 1.

Where Samples of the present invention were administered, the Amphotericin B concentration in kidney was less than the measurement limit in any case. Where Comparative Samples were administered, however, Amphotericin B was detected in a high concentration in all of the cases.

The fat emulsions of the present invention clearly achieve remarkable improvement in distribution of the drug into kidney (lowering in migration), as compared to conventionally known liposome preparations and fat emulsion preparation.

**Table 1**

| Amount of Amphotericin B Distributed into Kidney | |
|---|---|
| | Concentration in Kidney (µg/g) |
| Sample 1 of This Invention | Less than quantitative limit (<0.1) |
| Sample 2 of This Invention | Less than quantitative limit (<0.1) |
| Comparative Sample 1 | 1.4 ± 0.1 |
| Comparative Sample 2 | 1.3 ± 0.3 |
| Comparative Sample 5 | 1.5 ± 0.4 |
| (average value ± standard error, n = 3) | |

### Test Example 4: Evaluation of Kidney Function

Using SD strain male rats (weighing about 250 g) as experimental animals, each of Samples of the present invention and Comparative Samples was intravenously administered through the tail vein. The dose was 1 mg/kg as Amphotericin B and the drug was administered every other 24 hours 3 times in total. 24 hours after the final administration, blood was collected from the cervical vein to obtain serum. The amount of blood urea nitrogen (BUN) used as an index of the kidney functions was determined using a commercially available assay kit. The results are shown in Table 2. For control, physiological saline was administered in the same manner and the thus obtained serum was used.

**Table 2**

| Serological Evaluation of Kidney Function | |
|---|---|
| | BUN (mg/dl) |
| Control | 14.7 ± 1.7 |
| Sample 1 of This Invention | 14.7 ± 1.2 |
| Sample 2 of This Invention | 16.5 ± 1.5 |
| Comparative Sample 1 | 29.2 ± 2.6 |
| Comparative Sample 2 | 29.9 ± 2.1 |
| Comparative Sample 5 | 37.8 ± 5.4 |
| (average value ± standard error, n = 3) | |

Where Samples of the present invention were administered, there was no difference in BUN concentration between Samples and Control in any cases, indicating that no disturbance was noted in kidney functions. However, where Comparative Samples were administered, markedly high BUN concentration was noted in all cases, indicating that disturbance in the kidney functions was noted. It is evident that in the fat emulsions of the present invention, remarkable improvement in disturbance of kidney functions can be achieved, as compared to conventionally known liposome preparations and fat emulsion preparation.

### Test Example 5: Change in blood concentration

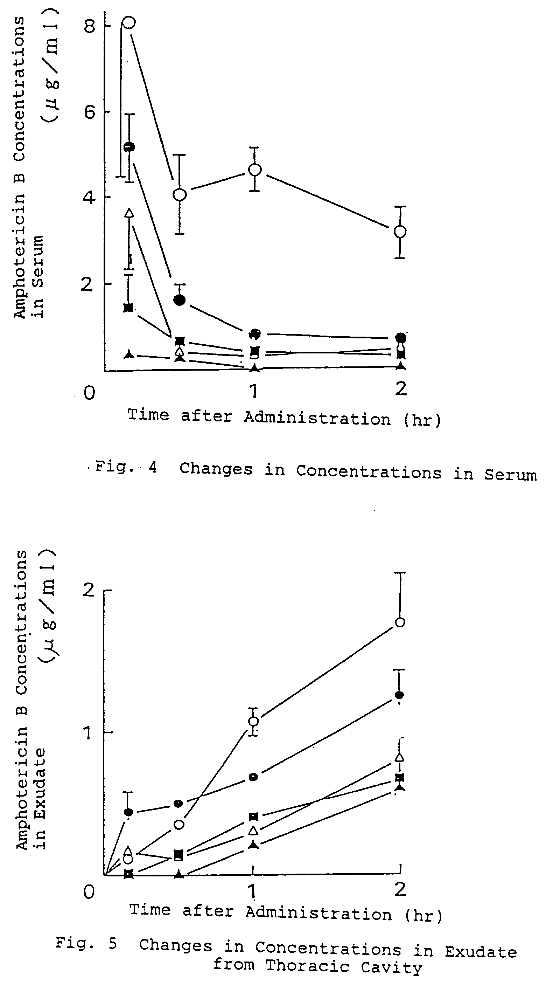

Using SD strain male rats (weighing about 250 g) as experimental animals, each of Samples of the present invention and Comparative Samples was intravenously administered through the tail vein. The dose administered was 1 mg/kg as Amphotericin B. After the administration, a small amount of blood was collected from the cervical vein to obtain serum. The concentration of Amphotericin B in kidney was determined by high performance liquid chromatography. The results are shown in Fig. 4.

Where Samples of the present invention were administered, the change of Amphotericin B concentration in serum was higher in any cases than in Comparative Samples. On the other hand, where any of Comparative Samples was administered, the concentration of Amphotericin B in serum decreased. It is evident that the fat emulsions of the present invention maintain remarkable blood concentration of Amphotericin B, as compared to conventionally known liposome preparations and fat emulsion preparation.

### Test Example 6: Distribution of Drug to Inflammatory Site

It is known that the site infected with fungi, etc. causes inflammation. Distribution of the drug into experimentally induced inflammatory site was evaluated in the model system.

Using SD strain male rats (weighing about 250 g) as experimental animals, 0.1 ml of 2% λ-carrageenin was intrathoracically administered to cause experimental pleurisy. Two hours and a half after, each of Samples of the present invention and Comparative Samples was intravenously administered through the tail vein. The dose administered was 1 mg/kg as Amphotericin B. After the administration, each animal was bled to death from the abdominal aorta in each time to obtain the exudate in the thoracic cavity. The concentration of Amphotericin B in the exudate was determined by high performance liquid chromatography. The results are shown in Fig. 5.

It was shown that where any of Samples of the present invention was administered, the change of Amphotericin B concentration in the exudate was higher than in any of Comparative Samples. It is evident that the fat emulsions of the present invention have the property of remarkably concentrating on the inflammatory site (infected site), as compared to conventionally known liposome preparations and fat emulsion preparation, and can achieve more effective and safer chemotherapy.

### Test Example 7: Measurement of particle diameter

The particle diameters of Sample 1 and Sample 2 of the present invention were evaluated using an apparatus for measuring dynamic light scattering particle diameter by laser light. As the result, the particle diameter of Sample 1 of the present invention showed about 20 to about 80 nm. Sample 1 did not contain particles having 1 µm or more. The particle diameter of Sample 2 of the present invention showed about 70 to about 200 nm. Sample 2 did not contain particles having 1 µm or more.

It is evident that the fat emulsion of the present invention comprises extremely fine and uniform emulsion particles. Furthermore the fat emulsion is free of particles of 1 µm or more which are problematic when administered intravenously. It is therefore evident that effective and safe chemotherapy can be attained.

### Test Example 8: Antifungal test (in vitro)

Candida bacteria (C. Albicans) was cultured in Sabouraud's medium and each of Samples of the present invention and Comparative Samples was added to the medium. The minimum inhibitory concentration of Amphotericin B for inhibiting the growth of Candida was determined to evaluate the antifungal activity of each sample. The results are shown in Table 3. Each sample showed the antifungal activity in an extremely trace concentration of Amphotericin B to inhibit the growth of Candida.

It was demonstrated that the fat emulsions of the present invention attain effective and safe chemotherapy without adversely affecting the antifungal activity possessed by Amphotericin B itself at all.

**Table 3**

| Antifungal Activity (in vitro) | |
|---|---|
| | Minimum Effective Concentration (µg/ml) |
| Sample 1 of This Invention | 0.03 or less |
| Sample 2 of This Invention | 0.16 or less |
| Comparative Sample 1 | 0.20 or less |
| Comparative Sample 3 | 0.14 or less |

### BRIEF DESCRIPTION OF THE DRAWINGS

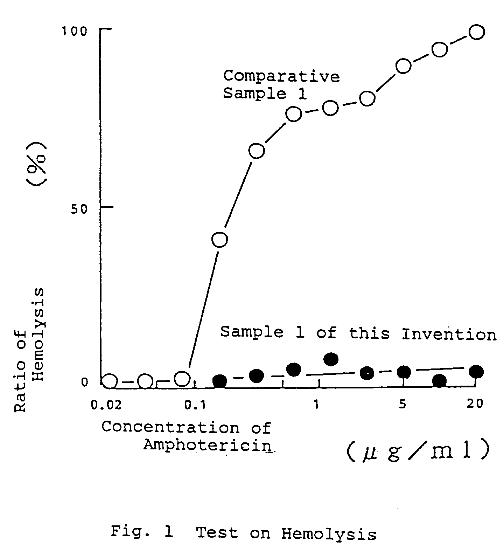

Fig. 1 shows the results of test of Sample 1 of the present invention and Comparative Sample 1 on hemolysis in vitro using rat erythrocyte. The abscissa designates concentration of Amphotericin B (µg/ml) and the ordinate designates hemolytic ratio by %, wherein the curve obtained by connecting black circles indicates Sample 1 of the present invention and the curve obtained by connecting white circles indicates Comparative Sample 1.

Fig. 2 shows the results of evaluation of toxicity of each sample in terms of survival rate of mice one hour after administration of each of Samples of the present invention and Comparative Samples. The abscissa designates the dose when calculated as Amphotericin B and the ordinate designates survival rate of mice one hour after the administration. The respective samples are shown on the curve obtained by connecting black circles, respectively.

Fig. 3 shows the results of evaluation of toxicity of each sample in terms of survival rate of mice 72 hours after administration of each of Samples of the present invention and Comparative Samples. The abscissa designates the dose when calculated as Amphotericin B and the ordinate designates survival rate of mice 72 hours after the administration. The respective samples are shown on the curve obtained by connecting black circles, respectively.

Fig. 4 shows the change of concentration of Amphotericin B in serum after administration of each of Samples of the present invention and Comparative Samples. The abscissa indicates progress of time (hour) after administration of each sample and the ordinate indicates concentration (µg/ml) of Amphotericin B in serum. The curves obtained by connecting white circles, black circles, black squares, black triangles and white triangles indicate the case of Sample 1 of the present invention, Sample 2 of the present invention, Comparative Sample 1, Comparative Sample 2 and Comparative Sample 5, respectively.

Fig. 5 shows the change in concentration of Amphotericin B in the exudate of the thoracic cavity in rats with experimentally induced pleurisy after administration of each of Samples of the present invention and Comparative Samples. The abscissa indicates progress of time (hour) after administration of each sample and the ordinate indicates concentration (µg/ml) of Amphotericin B in the exudate. The curves obtained by connecting white circles, black circles, black squares, black triangles and white triangles indicate the case of Sample 1 of the present invention, Sample 2 of the present invention, Comparative Sample 1, Comparative Sample 2 and Comparative Sample 5, respectively.

### INDUSTRIAL APPLICABILITY

As described above, the fat emulsion in accordance with the present invention can effect safe administration of polyene antifungal antibiotics such as Amphotericin B, etc. in an effective dose, as a pharmaceutical preparation.

## Claims (Claims for the following Contracting State(s): AT, BE, CH, DE, DK, FR, GB, IT, LI, LU, NL, SE)

1. A fat emulsion comprising (a), (b), (c) and (d) described below:
(a) 0.005 to 5% (w/v) of a polyene antifungal antibiotic based on the total weight,
(b) 0.5 to 30% (w/v) of a simple lipid based on the total weight,
(c) 0.15 to 2 times (weight ratio) of a phospholipid based on said simple lipid, and
(d) a suitable amount of water;
or a freeze-dried preparation thereof.

## Claims (Claims for the following Contracting State(s): ES, GR)

1. A process for preparing a fat emulsion comprising (a), (b), (c) and (d) described below:
(a) 0.005 to 5 % (w/v) of a polyene antifungal antibiotic based on the total weight,
(b) 0.5 to 30 % (w/v) of a simple lipid based on the total weight,
(c) 0.15 to 2 times (weight ratio) of a phospholipid based on said simple lipid, and
(d) a suitable amount of water,
which comprises the steps of mixing and emulsifying (a), (b), (c) and (d).

2. A process for preparing a freeze-dried preparation of a fat emulsion comprising (a), (b), (c) and (d) described below :
(a) 0.005 to 5 % (w/v) of a polyene antifungal antibiotic based on the total weight,
(b) 0.5 to 30 % (w/v) of a simple lipid based on the total weight,
(c) 0.15 to 2 times (weight ration) of a phospholipid based on said simple lipid, and
(d) a suitable amount of water,
which comprises the steps of mixing, emulsifying and freeze-drying (a), (b), (c) and (d).

## Patentansprüche (Patentansprüche für folgende(n) Vertragsstaat(en): AT, BE, CH, DE, DK, FR, GB, IT, LI, LU, NL, SE)

1. Fettemulsion mit den unten beschriebenen (a), (b), (c) und (d):
(a) 0,005 bis 5% (Gew./Vol.) eines pilzbekämpfenden Antibiotikums mit Polyen auf Basis des Gesamtgewichts,
(b) 0,5 bis 30% (Gew./Vol.) eines einfachen Lipids auf Basis des Gesamtgewichts,
(c) das 0,15- bis 2-fache (Gewichtsverhältnis) eines Phospholipids auf Basis des einfachen Lipids, und
(d) eine geeignete Menge an Wasser;
oder ein gefriergetrocknetes Präparat davon.

## Patentansprüche (Patentansprüche für folgende(n) Vertragsstaat(en): ES, GR)

1. Verfahren zum Herstellen einer Fettemulsion mit den unten beschriebenen (a), (b), (c) und (d):
(a) 0,005 bis 5% (Gew./Vol.) eines pilzbekämpfenden Antibiotikums mit Polyen auf Basis des Gesamtgewichts,
(b) 0,5 bis 30% (Gew./Vol.) eines einfachen Lipids auf Basis des Gesamtgewichts,
(c) das 0,15- bis 2-fache (Gewichtsverhältnis) eines Phospholipids auf Basis des einfachen Lipids, und
(d) eine geeignete Menge an Wasser,
welches die Verfahrensschritte des Mischens und Emulgierens von (a), (b), (c) und (d) umfaßt.

2. Verfahren zum Herstellen eines gefriergetrockneten Präparates einer Fettemulsion mit den unten beschriebenen (a), (b), (c) und (d):
(a) 0,005 bis 5% (Gew./Vol.) eines pilzbekämpfenden Antibiotikums mit Polyen auf Basis des Gesamtgewichts,
(b) 0,5 bis 30% (Gew./Vol.) eines einfachen Lipids auf Basis des Gesamtgewichts,
(c) das 0,15- bis 2-fache (Gewichtsverhältnis) eines Phospholipids auf Basis des einfachen Lipids, und
(d) eine geeignete Menge an Wasser,
welches die Verfahrensschritte des Mischens, Emulgierens und Gefriertrocknens von (a), (b), (c) und (d) umfaßt.

## Revendications (Revendications pour l'(les) Etat(s) contractant(s) suivant(s): AT, BE, CH, DE, DK, FR, GB, IT, LI, LU, NL, SE)

1. Emulsion de graisse comprenant les constituants (a), (b), (c) et (d) décrits ci-dessous :
(a) 0,005 à 5 % (p/v) d'un antibiotique antifongique de polyènes par rapport au poids total,
(b) 0,5 à 30 % (p/v) d'un lipide simple par rapport au poids total,
(c) un poids d'un phospholipide 0,15 à 2 fois plus élevé que celui de ce lipide simple, et
(d) une quantité d'eau appropriée; ou une préparation lyophilisée de celle-ci.

## Revendications (Revendications pour l'(les) Etat(s) contractant(s) suivant(s): ES, GR)

1. Procédé de préparation d'une émulsion de graisse comprenant les constituants (a), (b), (c) et (d) décrits ci-dessous :
(a) 0,005 à 5 % (p/v) d'un antibiotique antifongique de polyènes par rapport au poids total,
(b) 0,5 à 30 % (p/v) d'un lipide simple par rapport au poids total,
(c) un poids d'un phospholipide 0,15 à 2 fois plus élevé que celui du lipide simple, et
(d) une quantité d'eau appropriée,
qui comprend les étapes consistant à mélanger et à émulsionner (a), (b), (c) et (d).

2. Procédé de préparation d'une préparation lyophilisée d'une émulsion de graisse comprenant les constituants (a), (b), (c) et (d) décrits ci-dessous :
(a) 0,005 à 5 % (p/v) d'un antibiotique antifongique de polyènes par rapport au poids total,
(b) 0,5 à 30 % (p/v) d'un lipide simple par rapport au poids total,
(c) un poids d'un phospholipide 0,15 à 2 fois plus élevé que celui du lipide simple, et
(d) une quantité d'eau appropriée,
qui comprend les étapes consistant à mélanger, à émulsionner et à lyophiliser (a), (b), (c) et (d).
